# EUROPEAN PATENT APPLICATION

(11) **EP 4 494 579 A1**
(43) Date of publication of application: **22.01.2025**
(21) Application number: 23826188.7
(22) Date of filing: 12.06.2023
(51) Int. Cl.: A61B 17/3207

(54) **ROTABLATOR APPARATUS**

(30) Priority: 20.06.2022 CN 202210696567
(71) Applicant: Shanghai Microport Rotapace Medtech Co., Ltd., Shanghai 201203 (CN)
(72) Inventor: JI, Xiaofei, Shanghai 201203 (CN); CHANG, Zhaohua, Shanghai 201203 (CN); YUE, Bin, Shanghai 201203 (CN); YAO, Yingzhong, Shanghai 201203 (CN); LIU, Peifeng, Shanghai 201203 (CN)
(74) Representative: Marks & Clerk LLP
(86) International application number: PCT/CN2023/099612
(87) International publication number: WO 2023/246539

(57) **Abstract**

A rotablator apparatus (10), comprising a rotablator assembly (11), a drive shaft (12), a liquid passing pipe (14), and a liquid pumping part (15). A distal end of the drive shaft (12) is connected to the rotablator assembly (11) and is used for driving the rotablator assembly (11) to rotate. The liquid passing pipe (14) is sleeved on the drive shaft (12), and the liquid passing pipe (14) is provided with a liquid passing cavity (141). The liquid pumping member (15) is in transmission connection with the drive shaft (12), and driven by the drive shaft (12), can convey liquid to the far end of the liquid passing pipe (14) by means of the liquid passing cavity (141).

## Description

### CROSS-REFERENCE TO RELATED APPLICATIONS

This application claims priority to Chinese patent application No. 2022106965671, filed on June 20, 2022, the content of which is hereby incorporated by reference in its entirety.

### TECHNICAL FIELD

The present disclosure relates to the technical field of medical equipment, and in particular to a rotational atherectomy device.

### BACKGROUND

Atherosclerotic plaques are typically located in the vasculature of coronary arteries or peripheral arteries, and may have different characteristics depending on the texture of the plaques. Heavily calcified lesions need to be pretreated with an atherectomy device, of which the principle involves grinding up the vascular lesions by rotating a rotational atherectomy device at a high speed to ablate the calcified or fibrotic atherosclerotic plaques, thereby opening the blood vessels blocked by the plaques, obtaining an enlarged smooth vascular lumen, and thus facilitating subsequent stent implantation.

During the rotational atherectomy operation of the rotational atherectomy device, the rotary grinding head is mainly propelled to rotate at a high speed by the rotational catheter via the drive shaft, and is advanced forward to contact, grind up, and thereby ablate the lesions. Since heat will be generated during the process of grinding up lesions, in order to reduce the thermal damage to blood vessel tissues and blood, during the rotary grinding process a cooling solution (such as saline) needs to be infused to cool and flush the debris and microparticles that are ground off.

However, the rotational atherectomy device in the related art mainly delivers and infuses the cooling solution in the reservoir bag to the distal end of the catheter through a pressure bag or an infusion pump, playing a role in cooling and flushing the generated debris and microparticles, but cannot timely and effectively adjust the flow rate of the infusion of the cooling solution according to the actual rotational atherectomy situation.

### SUMMARY

Accordingly, a rotational atherectomy device is provided to solve the issue that the flow rate of the infusion of the cooling solution cannot be timely and effectively adjusted.

The present disclosure provided a rotational atherectomy device, including:
a rotary grinding assembly;
a drive shaft, a distal end of the drive shaft being connected to the rotary grinding assembly, and the drive shaft being configured to drive the rotary grinding assembly to rotate;
a liquid feeding pipe sleeved on the drive shaft and having a liquid feeding cavity; and
a pumping liquid member in transmission connection with the drive shaft, and the pumping liquid member being capable of delivering liquid toward a distal end of the liquid feeding pipe through the liquid feeding cavity when driven by the drive shaft.

### BRIEF DESCRIPTION OF THE DRAWINGS

To illustrate the technical solutions according to the embodiments of the present invention or in the prior art more clearly, the accompanying drawings for describing the embodiments or the prior art are introduced briefly in the following. Apparently, the accompanying drawings in the following description are only some embodiments of the present invention, and persons of ordinary skill in the art can derive other drawings from the accompanying drawings without creative efforts.
FIG. 1 is a schematic structural view of a rotational atherectomy device according to a first embodiment.
FIG. 2 is a schematic structural view of the cross-section of a pumping liquid member of the rotational atherectomy device according to the first embodiment.
FIG. 3 is a schematic structural view of an embodiment of the pumping liquid member of the rotational atherectomy device according to the first embodiment.
FIG. 4 is a schematic structural view of a rotational atherectomy device according to a second embodiment.
FIG. 5 is a schematic structural view of the cross-section of a pumping liquid member of the rotational atherectomy device according to the second embodiment.

### DETAILED DESCRIPTION

The embodiments of the present disclosure will be described in detail below, in order to make the above objects, features and advantages of the present disclosure more apparent and understandable. Numerous specific details are set forth in the following description in order to facilitate a thorough understanding of the present disclosure. However, the present disclosure can be implemented in many other ways than those describe herein, and similar modifications can be made by those skilled in the art without departing from the concept of the present disclosure, and thus the present disclosure is not limited to the embodiments disclosed below.

It should be noted that when one element is referred to as "fixed to" or "provided on" another element, it may be directly disposed on the other element or an intermediate element may exist. When one element is considered to be "connected to" another element, it may be directly connected to the other element or an intermediate element may co-exist.

The terms "vertical", "horizontal", "upper", "lower", "left", "right" and similar expressions used herein are for illustrative purposes only and do not represent the only implementation.

It should be understood that, the orientation or position relationships indicated by the terms "central", "longitudinal", "transverse", "length", "width", "thickness", "upper", "lower", "front", "back", "left", "right", "vertical", "horizontal", "top", "bottom", "inner", "outer", "clockwise", "counterclockwise", "axial", "radial", "circumferential", and the like are based on the orientation or position relationships shown in the accompanying drawings and are intended to facilitate the description of the present disclosure and simplify the description only, rather than indicating or implying that the apparatus or element referred to must have a particular orientation or be constructed and operated in a particular orientation, and therefore are not to be interpreted as limiting the present disclosure.

In the embodiment of the present application, the proximal end is the end of the medical device that is close to the operator (such as a doctor), and the distal end is the end of the medical device away from the operator.

The present disclosure provides a rotational atherectomy device, including:
a rotary grinding assembly;
a drive shaft, a distal end of the drive shaft being connected to the rotary grinding assembly, and the drive shaft being configured to drive the rotary grinding assembly to rotate;
a liquid feeding pipe sleeved on the drive shaft and having a liquid feeding cavity; and
a pumping liquid member in transmission connection with the drive shaft, and the pumping liquid member being capable of delivering liquid toward a distal end of the liquid feeding pipe through the liquid feeding cavity when driven by the drive shaft.

In one of the embodiments, the drive shaft extends through the liquid feeding cavity, and the pumping liquid member is coaxially provided on the drive shaft.

In one of the embodiments, the pumping liquid member has at least one axial groove, the axial groove enables a peripheral side of the pumping liquid member to form at least one rotor blade, and the rotor blade is configured to deliver liquid toward the distal end of the liquid feeding pipe along the liquid feeding cavity when the pumping liquid member rotates.

In one of the embodiments, an outer diameter of the rotor blade gradually increases from a proximal end thereof to a distal end thereof.

In one of the embodiments, the liquid feeding pipe includes a guide cavity spaced apart from the liquid feeding cavity, and the drive shaft extends through the guide cavity and is in transmission connection with the pumping liquid member through a transmission mechanism.

In one of the embodiments, the transmission mechanism includes a gear transmission assembly, the gear transmission assembly includes a driving wheel and a driven wheel engaged with each other, the driving wheel is connected to the drive shaft, and the driven wheel is connected to the pumping liquid member.

In one of the embodiments, the gear transmission assembly includes at least one intermediate gear engaged between the driving wheel and the driven wheel.

In one of the embodiments, a transmission ratio between the driving wheel and the driven wheel is greater than 1.

In one of the embodiments, wherein a transmission ratio between the driving wheel and the driven wheel is in a range from 1.1:1 to 3: 1.

In one of the embodiments, the transmission mechanism includes a worm gear and a worm engaged with each other, the worm is coaxially connected to the drive shaft, and the pumping liquid member includes a plurality of rotor blades connected to the worm gear, when the worm drives the worm gear to rotate, the worm gear drives the plurality of rotor blades to rotate.

In one of the embodiments, the surface where the rotor blade is located are inclined to an axial direction of the pumping liquid member.

In one of the embodiments, a rotation axis of the pumping liquid member is parallel to and spaced apart from the drive shaft.

In one of the embodiments, the rotational atherectomy device further includes a liquid inlet pipeline in communication with the liquid feeding cavity.

In one of the embodiments, the rotational atherectomy device further includes a drive assembly, the drive assembly is connected to a proximal end of the drive shaft and is configured to drive the drive shaft to rotate.

In one of the embodiments, the drive assembly is an electric motor or a pneumatic motor.

In one of the embodiments, the rotational atherectomy device further includes a guide wire extending through the drive shaft and the rotary grinding assembly, wherein the drive shaft and the rotary grinding assembly can move axially along the guide wire and rotate around the guide wire.

In one of the embodiments, the rotary grinding assembly includes a rotary grinding head, at least a portion of a surface of the rotary grinding head is covered with abrasive particles.

In one of the embodiments, the abrasive particles include diamond particles.

### First Embodiment

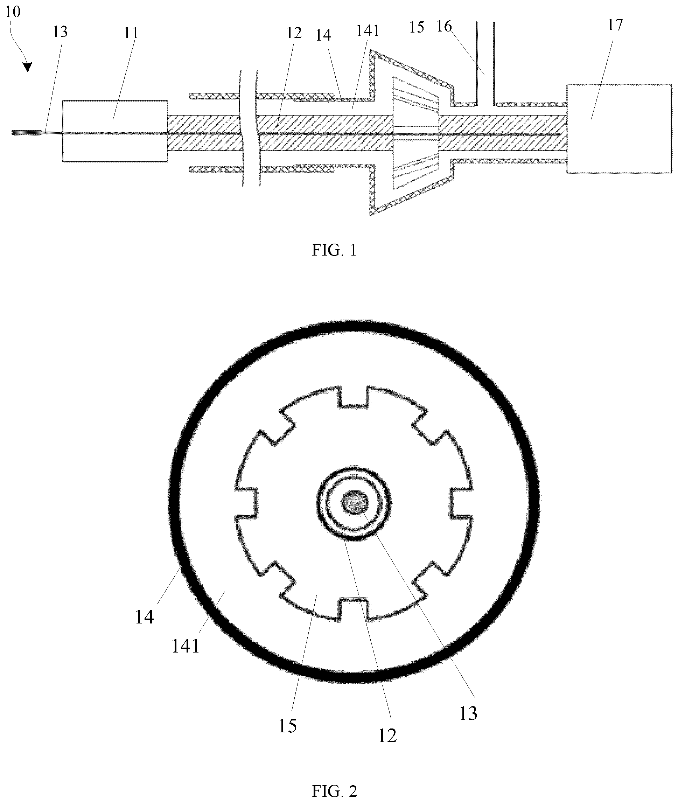

Referring to FIG. 1, a rotational atherectomy device 10 includes a rotary grinding assembly 11, a drive shaft 12, a guide wire 13, a liquid feeding pipe 14, a pumping liquid member 15, a liquid inlet pipeline 16, and a drive assembly 17.

The rotary grinding assembly 11 is connected to a distal end of the drive shaft 12, and the guide wire 13 extends through the rotary grinding assembly 11 and the drive shaft 12. The drive shaft 12 and the rotary grinding assembly 11 can move axially along the guide wire 13, and the drive shaft 12 and the rotary grinding assembly 11 can rotate around the guide wire 13. In some embodiments, when performing the rotational atherectomy operation, the drive shaft 12 and the rotary grinding assembly 11 can be operated to move back and forth along the guide wire 13. The "move axially" refers to moving toward the distal and proximal ends along the guide wire 13 to perform a rotational atherectomy on the position where the lumen (e.g., a blood vessel) needs the rotational atherectomy, so as to unclog the lumen.

In some embodiments, the rotary grinding assembly 11 includes a rotary grinding head, and at least a portion of the surface of the rotary grinding head is covered with abrasive particles. The abrasive particles include, but are not limited to, diamond particles, such that a good rotary grinding effect can be obtained during the rotation of the rotary grinding head along with the drive shaft 12.

It should be noted that, when the rotary grinding assembly 11 performs the rotational atherectomy, the guide wire 13 is not an essential structure, which can be omitted.

The liquid inlet line 16 is configured to input a cooling solution, the cooling solution includes, but is not limited to saline.

Hereinafter, for the convenience of description, the cooling solution is saline as an example.

The liquid inlet pipeline 16 is in communication with the liquid feeding pipe 14. The saline is delivered to the distal end through the liquid feeding cavity 141 of the liquid feeding pipe 14 to flow out, so as to flush the microparticles generated by the rotary grinding and take away the heat generated by the rotary grinding, thereby reducing the temperature rise caused by the rotation of the rotary grinding assembly 11 and the resulting thermal damage to blood vessel tissues and blood, and thus avoiding complications such as slow blood flow/no reflow.

The pumping liquid member 15 is an element capable of pumping liquid. Specifically, the pumping liquid member 15 is in transmission connection with the drive shaft 12, such that the pumping liquid member 15 can be driven by the drive shaft 12 to rotate, so as to pump liquid.

Specifically, the drive assembly 17 is connected to the drive shaft 12. Driven by the drive assembly 17, the drive shaft 12 drives the pumping liquid member 15 and the rotary grinding assembly 11 to rotate. In this embodiment, the pumping liquid member 15 is coaxially provided on the drive shaft 12, so that when the drive shaft 12 is driven by the drive assembly 17 to rotate, the pumping liquid member 15 can rotate along with the drive shaft 12. It should be understood that, the flow rate of saline can be controlled by controlling the rotation speed of the pumping liquid member 15. Since the power sources of the pumping liquid member 15 and the drive shaft 12 are both the drive assembly 17, the rotational atherectomy device 10 can change the amount of saline delivered to the distal end of the rotational atherectomy device 10 by changing the rotation speed of the drive shaft 12. Specifically, since the pumping liquid member 15 is coaxially provided on the drive shaft 12, as the rotation speed of the drive shaft 12 increases, the pumping liquid member 15 increases the amount of saline delivered and infused at the distal end, thereby increasing the flow rate of saline to flush the debris and microparticles generated during the rotary grinding of the rotary grinding assembly 11, and reduce the temperature rise caused by the rotation of the rotary grinding assembly 11 and the resulting thermal damage caused to blood vessel tissue and blood, and thus avoid complications such as slow blood flow/no reflow.

It should be noted that, as shown in FIG. 2, since the pumping liquid member 15 is coaxially provided on the drive shaft 12 and rotatably provided in the liquid feeding pipe 14, a liquid feeding cavity 141 is formed between an outer wall of the drive shaft 12 and an inner wall of the liquid feeding pipe 14. As such, when the drive shaft 12 drives the pumping liquid member 15 to rotate, the liquid feeding cavity 141 can not only deliver saline to the distal end of the rotational atherectomy device 10 to wash away the debris generated during the rotary grinding, but also take away the heat generated during the rotation of the drive shaft 12 to cool the drive shaft 12, thereby reducing the temperature rise caused by the rotation of the rotary grinding assembly 11 and the resulting thermal damage to blood vessel tissues and blood, and thus avoiding complications such as slow blood flow/no reflow.

In an embodiment where the guide wire 13 extends through the drive shaft 12 of the rotational atherectomy device 10, during the rotation of the pumping liquid member 15 with the drive shaft 12, a rotation axis of the pumping liquid member 15 coincides with the guide wire 13. In other words, the pumping liquid member 15 rotates about the guide wire 13. The rotation axis of the pumping liquid member 15 is an axis around which the pumping liquid member 15 rotates.

It should be noted that, the liquid feeding pipe 14 may be a single pipe piece or may be formed by combining a plurality of pipe pieces. For example, referring to FIG. 1, a diameter of a portion of the liquid feeding pipe 14 corresponding to the pumping liquid member 15 may be set to be larger than that of the other portions, so as to facilitate the mounting of the pumping liquid member 15.

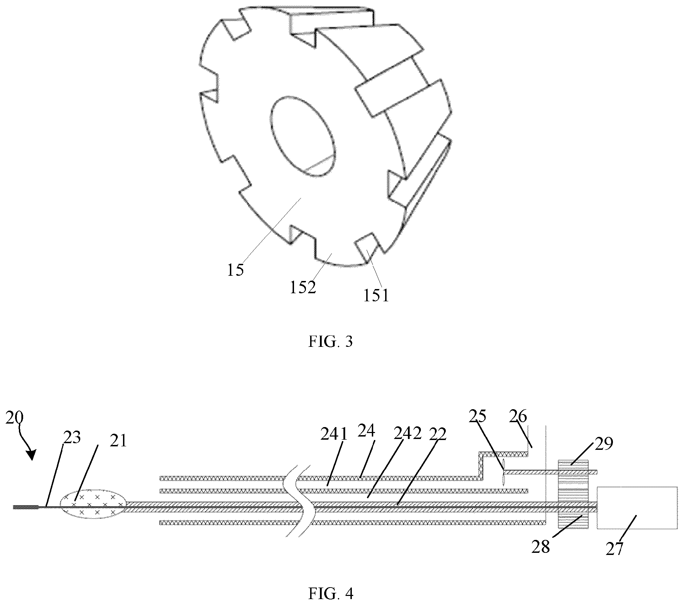

As shown in FIGS. 2 and 3, the pumping liquid member 15 has at least one axial groove 151, which enables a peripheral side of the pumping liquid member 15 to form at least one rotor blade 152. During the rotation of the pumping liquid member 15, the rotor blade 152 pushes the saline along the liquid feeding cavity 141 toward the distal end of the liquid feeding pipe 14, and finally enables the saline to be discharged from the distal end of the liquid feeding pipe 14. In this embodiment, the distal end of the liquid feeding pipe 14 is located at the position of the rotary grinding. Specifically, the debris generated during the rotary grinding of the rotary grinding assembly 11 can be flushed by the saline output from the liquid feeding pipe 14.

Further, an outer diameter of the rotor blade 152 gradually increases from the proximal end to the distal end. In this embodiment, the saline can flow through the surface of the rotor blade 152 via the liquid feeding cavity 141. The larger the diameter of the distal end of the rotor blade 152 of the pumping liquid member 15, the greater the rotation linear speed, and the higher flow rate of the driving liquid. According to the Bernoulli's principle, the liquid pressure is lower where the flow rate at the distal end of the pumping liquid member 15 is larger, and the liquid pressure is higher where the flow rate at the proximal end of the pumping liquid member 15 is lower. Under the action of the pressure difference, the liquid is accelerated to flow from the proximal end to the distal end. Therefore, the use of such pumping liquid member 15 can improve the delivery performance of saline, and facilitate the delivery of saline along the liquid feeding pipe 14 to the rotary grinding position at the distal end.

It should be particularly noted that, with the structure of the pumping liquid member 15, regardless of whether the pumping liquid member 15 rotates forward or reversely, the saline always flows toward the distal end along the liquid feeding pipe 14. Therefore, during the rotary grinding process, the flow rate of the saline increases with the increase of the rotation speed of the worm gear, and is not affected by the rotation direction of the pumping liquid member 15.

As shown in FIG. 3, the pumping liquid member 15 has eight rotor blades 152. In some embodiments, the pumping liquid member 15 has more rotor blades 152, for example, 9 or 10. The number of rotor blades 152 of the pumping liquid member 15 is not limited herein.

The rotation speeds of the drive shaft 12, the rotary grinding assembly 11 and the pumping liquid member 15 that are of the rotational atherectomy device 10 are the same, and the rotation speed is obtained from the drive assembly 17. In this embodiment, the drive assembly 17 may be an electric motor or a pneumatic motor, as long as it can drive the drive shaft 12 to rotate.

In some embodiments, the rotation speed of the rotary grinding assembly 11 driven by the drive assembly 17 is in a range from 1000 rad/min to 200,000 rad/min, such as 1,000 rad/min, 2,000 rad/min, 5,000 rad/min, 10,000 rad/min, 15,000 rad/min, 150,000 rad/min, 170,000 rad/min, or 200,000 rad/min. When the pumping liquid member 15 rotates, the flow rate of saline delivered from the liquid feeding cavity 141 of the liquid feeding pipe 14 to the distal end of the rotational atherectomy device 10 is in a range from 0.1 ml/min to 100 ml/min. Preferably, when the driving assembly 17 drives the rotary grinding assembly 11 to rotate, the rotation speed of the rotary grinding assembly 11 is 200,000 rad/min, and the flow rate of saline generated by the rotation of the pumping liquid member 15 is 50 ml/min, so that the effect of the rotary grinding performed by the rotary grinding assembly 11 is ensured, and the saline is effectively used to flush the debris generated by the rotary grinding.

### Second Embodiment

As shown in FIG. 4, the rotational atherectomy device 20 according to the second embodiment is similar to the rotational atherectomy device 10 according to the first embodiment, and the rotational atherectomy device 20 includes a rotary grinding assembly 21, a drive shaft 22, a guide wire 23, a liquid feeding pipe 24, a pumping liquid member 25, a liquid inlet pipeline 26, and a drive assembly 27.

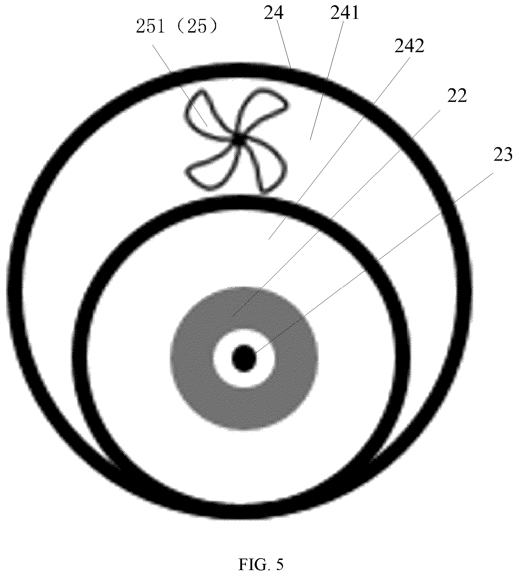

The guide wire 23 is not an essential structure. In the embodiment in which the rotational atherectomy device 20 includes the guide wire 23, as shown in FIGS. 4 and 5, the guide wire 23 extends through the rotary grinding assembly 21 and the drive shaft 22. The drive shaft 22 and the rotary grinding assembly 21 can move axially along the guide wire 23, and the drive shaft 22 and the rotary grinding assembly 21 can rotate around the guide wire 23.

Compared with the first embodiment, in the rotational atherectomy device 20 according to the second embodiment, the pumping liquid member 25 and the drive shaft 22 are not coaxial. Specifically, the rotational atherectomy device 20 includes a transmission mechanism, through which the pumping liquid member 25 is in transmission connection with the drive shaft 22. With such structure, when the drive shaft 22 rotates, the drive shaft 22 can drive the pumping liquid member 25 to rotate through the transmission mechanism. When the rotary grinding head located at the distal end of the drive shaft 22 performs rotary grinding operation, the pumping liquid member 25 can deliver saline toward the distal end through the liquid feeding pipe 24 to the rotary grinding position.

Since the pumping liquid member 25 is in transmission connection with the drive shaft 22 by the transmission mechanism, when the drive shaft 22 accelerates, the pumping liquid member 25 also accelerates together, so as to increase the flow rate of saline flushing the debris and microparticles, thereby reducing the temperature rise caused by the high-speed rotation of the rotary grinding head and the resulting thermal damage to blood vessel tissues and blood, and thus avoiding complications such as slow blood flow/no reflow.

In some embodiments, the transmission mechanism includes, but is not limited to, a gear transmission assembly. For example, the gear transmission assembly includes a driving wheel 28 and a driven wheel 29 that engage with each other. The driving wheel 28 is connected to the drive shaft 22, and the driven wheel 29 is connected to the pumping liquid member 25. Preferably, the driven wheel 29 is connected to the pumping liquid member 25 through a transmission shaft. When the drive shaft 22 rotates, the driving wheel 28 is engaged with and drives the driven wheel 29 to rotate, so that the driven wheel 29 drives the pumping liquid member 25 to rotate through the transmission shaft.

Further, the transmission ratio between the driving wheel 28 and the driven wheel 29 is greater than 1, so that when the drive shaft 22 rotates, the driven wheel 29 can transmit as much torque as possible to the pumping liquid member 25 via the transmission shaft, so that the pump liquid member 25 has enough thrust force to deliver the saline through the liquid feeding pipe 24 to the rotary grinding position. The transmission ratio between the driving wheel 28 and the driven wheel 29 may be specifically in a range from 1.1:1 to 3: 1.

In some embodiments, the transmission ratio between the driving wheel 28 and the driven wheel 29 may also be less than or equal to 1. When the transmission ratio between the driving wheel 28 and the driven wheel 29 is equal to 1, the pumping liquid member 25 always maintains the same rotation speed as the drive shaft 22 during the rotation of the drive shaft 22. When the transmission ratio between the driving wheel 28 and the driven wheel 29 is less than 1, during the rotation of the drive shaft 22, the pumping liquid member 25 can inject the saline into the liquid feeding pipe 24 at a rotation speed greater than that of the drive shaft 22, thereby effectively increasing the flow rate of saline for flushing the debris and microparticles.

It should be noted that, the gear transmission assembly is not limited to a structure in which the driving wheel 28 and the driven wheel 29 are engaged. In some embodiments, the gear transmission assembly further includes at least one intermediate gear, and the intermediate gear is engaged between the driving wheel 28 and the driven wheel 29, such that there is a suitable transmission ratio between the driving wheel 28 and the driven wheel 29.

In some embodiments, the transmission mechanism may be a worm gear-worm transmission structure. Specifically, the transmission mechanism includes a worm gear and a worm that are engaged with each other. The worm is coaxially connected to the drive shaft 22, so that when the drive shaft 22 rotates, the worm drives the worm gear to rotate. In this embodiment, the torque of the drive shaft 22 can be transmitted to the pumping liquid member 25 by means of the worm of the transmission mechanism driving the worm gear, so as to realize the transmission connection between the drive shaft 22 and the pumping liquid member 25. Specifically, the pumping liquid member 25 includes a plurality of rotor blades 251, and the plurality of rotor blades 251 are connected to the worm gear. When the worm drives the worm gear to rotate, the rotor blades 251 located on the worm gear also rotate together, thereby realizing the delivery of saline to the rotary grinding position through the liquid feeding pipe 24, and flushing the debris generated in the rotary grinding process as an example.

It should be noted that, in the embodiment in which the transmission mechanism is adopted to make the pumping liquid member 25 rotate with the drive shaft 22, the rotation axis of the pumping liquid member 25 does not coincide with the drive shaft 22. For example, they are parallel to and spaced apart from each other. Correspondingly, the liquid feeding cavity 241 for providing the pumping liquid member 25 does not coincide with the drive shaft 22. Specifically, an independent liquid feeding cavity 241 is formed by the liquid feeding pipe 24 sleeved outside the drive shaft 22. Particularly, the liquid feeding pipe 24 includes at least two pipe cavities separated from each other. Taking the liquid feeding pipe 24 including two pipe cavities as an example, as shown in FIG. 5, one of the two pipe cavities is used as the liquid feeding cavity 241 for delivering saline, and the other one can be used as the guide cavity 242 for the drive shaft 22, and the drive shaft 22 has good stability when moving in the axial direction under the guidance of the guide cavity 242.

As shown in FIG. 5, the pumping liquid member 25 includes a plurality of oblique rotor blades 251. Specifically, the surfaces where the rotor blades 251 are located are inclined rather than perpendicular to the axial direction of the pumping liquid member 25, similar to the blades of a fan. The rotor blades 251, when rotates, can increase the flow rate of the liquid in the liquid feeding cavity 241. In this embodiment, since the flow direction of the saline driven by the pumping liquid member 25 is related to the rotation direction of the pumping liquid member 25, the flow direction of the saline can be adjusted by controlling the rotation direction of the drive shaft 22.

The number of the rotor blades 251 of the pumping liquid member 25 may be specifically four, or may be two or three, and the number of the rotor blades 251 is not limited herein.

In some embodiments, the rotation speed of the drive shaft 22 is 200,000 rad/min, and when the drive shaft 22 drives the pumping liquid member 25 to rotate through the transmission mechanism, the rotation speed of the pumping liquid member 25 is 20,000 rad/min, and the flow rate of saline generated by the rotation of the pumping liquid member 25 is 30 ml/min. It should be noted that, in some other embodiments, the rotation speed of the drive shaft 22 and the rotation speed of the pumping liquid member 25 may be other values. For example, when the drive shaft 22 drives the pump liquid member 25 to rotate by a transmission mechanism, the rotation speed of the drive shaft 22 is in a range from 150,000 rad/min to 250,000 rad/min, and the rotation speed of the pumping liquid member 25 is 1/10 to 1/2 of the rotation speed of the drive shaft 22, which is not limited herein.

In the second embodiment, the drive assembly 27 may be an electric motor or a pneumatic motor, which is not limited herein.

It should be noted that, in the embodiments of the present disclosure, the terms "first" and "second" are used for descriptive purposes only, which cannot be construed as indicating or implying a relative importance, or implicitly specifying the number of the indicated technical features. Thus, the features defined with "first" and "second" may explicitly or implicitly include at least one feature. In the description of the present disclosure, "a plurality of" means two or more, such as two or three, unless otherwise defined explicitly and specifically.

In the present disclosure, unless otherwise specified and defined explicitly, the terms "install", "connect", "join", and "fix" should be understood in a broad sense. For example, unless otherwise defined explicitly, they may refer to a fixed connection, a detachable connection, or an integral connection, may refer to a mechanical connection or electrical connection, and may refer to a direct connection, an indirect connection via an intermediate medium, an internal connection between two elements, or interaction between two elements. Those of ordinary skill in the art can understand specific meanings of these terms in the present disclosure according to specific situations.

In the present disclosure, unless otherwise specified and defined explicitly, the expression a first feature being "on" or "under" a second feature may be the case that the first feature is in direct contact with the second feature, or the first feature is in indirect contact with the second feature via an intermediate medium.

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present disclosure.

The above-described embodiments are only several implementations of the present disclosure, and the descriptions are relatively specific and detailed, but they should not be construed as limiting the scope of the present disclosure. It should be understood by those of ordinary skill in the art that various modifications and improvements can be made without departing from the concept of the present disclosure, and all fall within the protection scope of the present disclosure. Therefore, the patent protection of the present disclosure shall be defined by the appended claims.

## Claims

1. A rotational atherectomy device, comprising:
a rotary grinding assembly;
a drive shaft, a distal end of the drive shaft being connected to the rotary grinding assembly, and the drive shaft being configured to drive the rotary grinding assembly to rotate;
a liquid feeding pipe sleeved on the drive shaft and having a liquid feeding cavity; and
a pumping liquid member in transmission connection with the drive shaft, and the pumping liquid member being capable of delivering liquid toward a distal end of the liquid feeding pipe through the liquid feeding cavity when driven by the drive shaft.

2. The rotational atherectomy device according to claim 1, wherein the drive shaft extends through the liquid feeding cavity, and the pumping liquid member is coaxially provided on the drive shaft.

3. The rotational atherectomy device according to claim 2, wherein the pumping liquid member has at least one axial groove, the axial groove enables a peripheral side of the pumping liquid member to form at least one rotor blade, and the rotor blade is configured to deliver liquid toward the distal end of the liquid feeding pipe along the liquid feeding cavity when the pumping liquid member rotates.

4. The rotational atherectomy device according to claim 3, wherein an outer diameter of the rotor blade gradually increases from a proximal end thereof to a distal end thereof.

5. The rotational atherectomy device according to claim 1, wherein the liquid feeding pipe comprises a guide cavity spaced apart from the liquid feeding cavity, and the drive shaft extends through the guide cavity and is in transmission connection with the pumping liquid member through a transmission mechanism.

6. The rotational atherectomy device according to claim 5, wherein the transmission mechanism comprises a gear transmission assembly, and the gear transmission assembly comprises a driving wheel and a driven wheel engaged with each other, the driving wheel is connected to the drive shaft, and the driven wheel is connected to the pumping liquid member.

7. The rotational atherectomy device according to claim 6, wherein the gear transmission assembly comprises at least one intermediate gear engaged between the driving wheel and the driven wheel.

8. The rotational atherectomy device according to claim 6, wherein a transmission ratio between the driving wheel and the driven wheel is greater than or equal to 1.

9. The rotational atherectomy device according to claim 6, wherein a transmission ratio between the driving wheel and the driven wheel is in a range from 1.1:1 to 3: 1.

10. The rotational atherectomy device according to claim 5, wherein the transmission mechanism comprises a worm gear and a worm engaged with each other, the worm is coaxially connected to the drive shaft, and the pumping liquid member comprises a plurality of rotor blades connected to the worm gear, when the worm drives the worm gear to rotate, the worm gear drives the plurality of rotor blades to rotate.

11. The rotational atherectomy device according to claim 10, wherein a surface where the rotor blade is located are inclined to an axial direction of the pumping liquid member.

12. The rotational atherectomy device according to claim 5, wherein a rotation axis of the pumping liquid member is parallel to and spaced apart from the drive shaft.

13. The rotational atherectomy device according to claim 1, further comprising a liquid inlet pipeline in communication with the liquid feeding cavity.

14. The rotational atherectomy device according to claim 1, further comprising a drive assembly, wherein the drive assembly is connected to a proximal end of the drive shaft and configured to drive the drive shaft to rotate.

15. The rotational atherectomy device according to claim 14, wherein the drive assembly is an electric motor or a pneumatic motor.

16. The rotational atherectomy device of claim 1, further comprising a guide wire extending through the drive shaft and the rotary grinding assembly, wherein the drive shaft and the rotary grinding assembly are capable of moving axially along the guide wire and rotating around the guide wire.

17. The rotational atherectomy device of claim 1, wherein the rotary grinding assembly comprises a rotary grinding head, at least a portion of a surface of the rotary grinding head is covered with abrasive particles.

18. The rotational atherectomy device of claim 17, wherein the abrasive particles comprise diamond particles.
